Europäisches Patentamt

European Patent Office

Office européen des brevets

(11) Publication number: **0 247 746**
**A1**

# EUROPEAN PATENT APPLICATION

(21) Application number: **87304072.9**

(22) Date of filing: **07.05.87**

(51) Int. Cl.⁴: **A61B 17/36**

(30) Priority: **27.05.86 JP 79804/86 U**

(43) Date of publication of application:
**02.12.87 Bulletin 87/49**

(84) Designated Contracting States:
**DE FR GB IT NL SE**

(71) Applicant: **SUMITOMO ELECTRIC INDUSTRIES LIMITED**
**No. 15, Kitahama 5-chome Higashi-ku**
**Osaka-shi Osaka 541(JP)**

(72) Inventor: **Iwai, Tohru**
**1-3, Shimaya 1-chome Konohana-ku**
**Osaka(JP)**
Inventor: **Sogawa, Ichiro**
**1-3, Shimaya 1-chome Konohana-ku**
**Osaka(JP)**

(74) Representative: **Rackham, Stephen Neil et al**
**GILL JENNINGS & EVERY 53-64 Chancery Lane**
**London WC2A 1HN(GB)**

(54) **Laser catheter.**

(57) A laser catheter for directing a laser beam (5) to an affected part such as an obstruction inside a living body includes a catheter tube (1) having a smooth inner surface and a window member (3) attached to one end. A liquid injection port (2) is provided at the one end of the catheter tube (1). A transparent liquid (4) is injected into the catheter tube (1) via the liquid injection port (2). The liquid has a greater refractive index than that of the material that forms the catheter tube (1). Thus, the catheter tube (1) acts as a clad optical fibre with its cladding formed by the catheter tube (1) and its core formed by the liquid (4). The laser beam is directed through the catheter (1) to an affected part in order to destroy it. The overall outer diameter of such a catheter (1) is much smaller than conventional devices and it is therefore possible to insert it into relatively thin blood vessels. In addition, since the catheter (1) is very flexible its distal end can reach a remote affected part of a curved blood vessel.

Fig. 1

## LASER CATHETER

The present invention relates to a laser catheter for use in leading and applying a laser beam to an affected part of a patient's body such as a closed or constricted part of a blood vessel in order to remove obstacles from it.

Thrombosis and other similar pathological conditions generally occur as a result of the occurrence of obstacles such as fat deposits, atheroma, calcium deposits and coagulation in blood vessels.

To remove such obstacles from blood vessels, it is conventional practice to adopt methods such as a drug treatment in which obstacles are removed using a resolvent, a cutting method in which affected parts of blood vessels are cut off, and a balloon inflating method.

However, these conventional methods have the following disadvantages. With the drug treatment method, it is relatively difficult to remove obstacles. The cutting method necessitates a relatively large-scale surgical operation. In the balloon inflating method, a balloon is inflated to gradually apply pressure to the side portions of the constricted part of a blood vessel so as to increase the diameter of this part of the blood vessel. However, this method cannot be used for a completely closed blood vessel.

A new therapeutic method has also been devised in which a laser beam is applied to an affected part to destroy and remove the obstacle. One example of this type of prior art is the "Laser Catheter with Fixed Focal Point" disclosed in JP-A-85-176641.

According to this prior art, a multiplicity of optical fibres are coaxially and annularly disposed around a catheter tube having at least one bore, and after this catheter tube has been inserted into a blood vessel so that its distal end reaches an affected part, e.g., a closed part, a therapeutic liquid is injected through the bore of the catheter to clean or dissolve the closed part and, at the same time, the closed part is irradiated with laser beam which is led through the optical fibres to destroy the obstruction.

This arrangement suffers, however, from the following disadvantages. Since a plurality of optical fibres are disposed around the catheter tube, the overall outer diameter of the catheter is large, so that it is difficult to insert the catheter into relatively thin blood vessels, and the flexibility of the catheter tube is lowered correspondingly, which means that it is difficult to lead the distal end of the catheter tube to an affected part in a blood vessel.

According to this invention a laser catheter for directing a laser beam to an affected part inside a living body comprises:-

a catheter tube having a smooth inner surface;

a window aligned with the catheter tube and attached to one end of it;

a liquid injection port at the one end of the catheter tube for injecting liquid into it; and,

a transparent liquid injected into the catheter tube from the liquid injection port, the liquid having a greater refractive index than that of the catheter tube.

In accordance with the present invention, the laser catheter is arranged so that the catheter forms a clad optical fibre having its cladding formed by the catheter tube and its core formed by the liquid injected into the inside of the tube, and laser beam travels through to core to an affected part so as to destroy it. Further the catheter also provides a liquid carrying passage to enable a therapeutic liquid to be introduced and removed to enable debris to be removed. Accordingly, there is no need to dispose optical fibres around a separate catheter tube as in the prior art, which means that the overall outer diameter of the catheter is much smaller, and it is therefore possible to insert the laser catheter into relatively thin blood vessels. Its applicability is thus increased. In addition, since the flexibility of the catheter is improved correspondingly, the distal end of the catheter tube can reach relatively remote affected parts in a curved blood vessel, resulting in a further increase in the applicability.

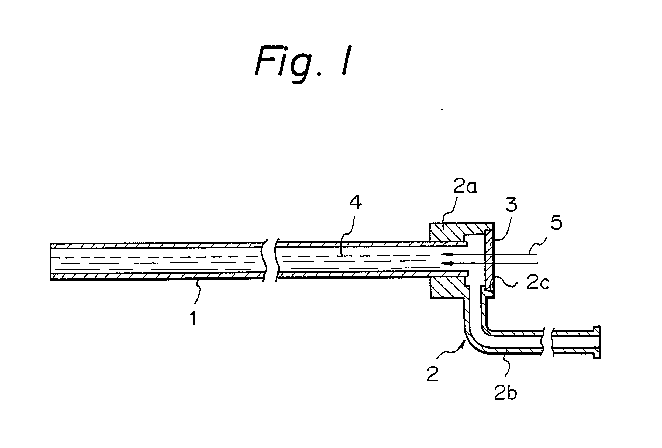

A particular example of a laser catheter in accordance with this invention will now be described with reference to the accompanying drawing, Figure 1, which is a longitudinal section.

Referring to Figure 1, the reference numeral 1 denotes a catheter tube which serves as a cladding of an optical fibre arranged by the present invention. When visible light, which is obtained from an argon laser or the like, is employed as laser beam (described later), the catheter tube 1 is made from a material, for example, tetrafluoroethylene-hexafluoropropylene copolymer, having a refractive index of 1.34, and the inner surface of the tube 1 is made smooth. It should be noted that the catheter tube 1 may also be made from a tetrafluoroethylene resin or a methacrylate resin containing fluorine in addition to the above-described material.

A liquid injection tube 2 which serves as a liquid injection port has a fitting portion 2a, a liquid injection portion 2b which is integral therewith and a recess 2c provided at the righthand (as viewed in the Figure) end of the fitting portion 2a. The liquid injection tube 2 is secured to the catheter tube 1 in such a manner that the fitting portion 2a is tightly fitted and bonded to the right-hand end of the catheter tube 1 so that no liquid leaks out.

A disk-shaped window plate 3 is made from a material, for example, glass, a transparent resin such as a methacrylate resin or a styrene resin, or a transparent crystal such as quartz or sapphire. The window plate 3 is fitted within the recess 2c provided at the right-hand end of the liquid injection tube 2 in such a manner that the plane of the plate 3 extends perpendicularly to the axial direction of the fitting portion 2a, and is tightly bonded to the recess so that no liquid leaks out.

The reference numeral 4 denotes a liquid which serves as a core of the optical fiber arranged by the present invention, the liquid being, for example, glycerin having a refractive index of 1.473. Glycerin is a substance which is also contained in the blood and can safely be injected into a blood vessel, provided that the amount of injected glycerin does not exceed about 2 g. Since the liquid 4 needs to serve as the core of the optical fiber, it must be a substance having a greater refractive index than that of the catheter tube 1 that serves as the cladding of the optical fiber. It will be convenient to employ as the liquid 4 a substance which satisfies the above-described precondition and which is also capable of cleaning or dissolving, for example, a closed part of a blood vessel, as in the case of the prior art. In this sense, the liquid 4 may need not only be glycerin but may also be a mixture of glycerin and a physiological saline or a isotonic dextrose solution, or other appropriate liquids. However, in this embodiment a physiological saline or a isotonic dextrose solution cannot be employed alone because in such a case the refractive index of the liquid 4 would be smaller than that of the catheter tube 1.

The following is a description of the way in which the above-described embodiment is actually used.

The catheter tube 1 is first inserted into a blood vessel from its left-hand end as viewed in Fig. 1 so that the end reaches the closed part of a blood vessel.

Then, a predetermined liquid 4 is injected through the injection portion 2b of the liquid injection tube 2 so as to fill the insides of the fitting portion 2a and the catheter tube 1.

Subsequently, visible laser beam 5, which is obtained from, for example, an argon laser, is applied to the inside of the catheter tube 1 through the window plate 3 and is led to the closed part of the blood vessel to destroy it. In this case, since the laser catheter per se constitutes one optical fiber having its cladding defined by the catheter tube 1 and its core defined by the liquid 4, the laser beam 5 is efficiently guided through the liquid 4 to reach the closed part. It should be noted that the laser beam which may be employed in the present invention is not limited to visible light and a laser beam in the near infrared region may also be employed.

According to this embodiment, it is theoretically possible to reduce the outer diameter of the laser catheter tube 1 to the same dimension as the outer diameter of a single ordinary thin resin tube. The laser catheter can therefore be inserted into a very thin blood vessel. In addition, the flexibility of the catheter tube 1 increases correspondingly, so that the distal end of the catheter can reach a closed part of a curved blood vessel that is relatively remote.

## Claims

1. A laser catheter for directing a laser beam to an affected part inside a living body, comprising:

a catheter tube (1) having a smooth inner surface;

a window (3) aligned with the catheter tube (1) and attached to one end of it;

a liquid injection port (2) at the one end of the catheter tube (1) for injecting liquid into it; and,

a transparent liquid (4) injected into the catheter tube (1) from the liquid injection port (2), the liquid (4) having a greater refractive index than that of the catheter tube (1).

2. A laser catheter according to Claim 1, wherein the catheter tube (1) is made from tetrafluoroethylene-hexafluoropropylene copolymer or tetrafluoroethylene resin or methacrylate resin containing fluorine.

3. A laser catheter according to Claim 1 or 2, wherein the liquid (4) is either glycerin or a mixture of glycerin and a physiological saline or an isotonic dextrose solution.

4. A laser catheter for removing obstacles from blood vessels comprising:-

a laser;

a transparent catheter tube (1) having a smooth inner surface;

a window (3) at one end of the catheter tube (1) aligned with the inside of it to admit light (5) from the laser into the inside of it;

a liquid injection port (2) at the one end of the catheter tube (1) for injecting liquid into it; and,

a transparent liquid (4) for injection into the catheter tube (1) via the liquid injection port (2), the transparent liquid (4) having a greater refractive index than that of the catheter tube (1).

**0 247 746**

5. A laser catheter according to Claim 4, wherein the laser is one operating in the visible region or in the near infrared region of the electromagnetic spectrum.

6. A laser catheter for surgical, therapeutic or diagnostic use comprising:-

a transparent catheter tube (1) having a smooth inner surface;

a window (3) at one end of the catheter tube (1) aligned with the inside of it to admit light (5) from the laser into the inside of it; and,

a liquid injection port (2) at the one end of the catheter tube (1) for injecting liquid into it.

# Fig. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 995 934 (G.NATH)<br>* Whole document * | 1-6 | A 61 B 17/36 |

-----

|  |  |  | TECHNICAL FIELDS SEARCHED (Int. Cl.4) |
|---|---|---|---|
|  |  |  | A 61 B<br>G 02 B<br>B 23 K |

The present search report has been drawn up for all claims

| Place of search | Date of completion of the search | Examiner |
|---|---|---|
| THE HAGUE | 19-08-1987 | NEILL M.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82